# EUROPEAN PATENT APPLICATION

(11) **EP 3 141 171 A1**
(43) Date of publication of application: **15.03.2017**
(21) Application number: 16160355.0
(22) Date of filing: 15.03.2016
(51) Int. Cl.: A47K 7/04, A46B 13/02, A46B 5/00

(54) **ELECTRIC CLEANING DEVICE**

(30) Priority: 10.09.2015 TW 104129921
(71) Applicant: Cal-Comp Electronics & Communications Company Limited, New Taipei City 22201 (TW); Kinpo Electronics, Inc., New Taipei City 22201 (TW)
(72) Inventor: TANG, Ching-Yi, 22201 New Taipei City (TW); KUO, Hsin-Ju, 22201 New Taipei City (TW); HSU, Cheng-Chih, 22201 New Taipei City (TW)
(74) Representative: Chamberlain, Alan James

(57) **Abstract**

An electric cleaning device is provided. The electric cleaning device includes a main body, a power source, a transmission shaft, a support stand, and a plurality of brush heads. The power source is disposed inside the main body. One end of the transmission shaft is connected to the power source, while the other end of the transmission shaft extends out of the main body and is connected to the support stand. The brush heads are flexibly assembled to the support stand and form an enclosed shape. In addition, the power source drives the brush heads to vibrate through the transmission shaft and the support stand.

## Description

### BACKGROUND OF THE DISCLOSURE

### 1. Field of the Disclosure

The disclosure relates to a cleaning device, and particularly relates to an electric facial cleaning device.

### 2. Description of Related Art

Facial cleaning is part of the daily routine. By cleaning the face, the dust and oil on the face can be washed away, and the skin pores may be prevented from being clogged. However, the foaming effect of the facial lotion is not desirable if it is only rubbed with bare hands. To solve this issue, there are already facial cleaning brushes on the market. The facial cleaning brushes function by driving the bristles disposed on the outside with a power mechanism, so as to vibrate the bristles to brush the face with the vibrating bristles. In this way, the facial cleanser may generate fine bubbles and gets into the skin deeply, so as to offer a desirable effect of cleaning along with the effects of exfoliation and skin revitalization.

However, the conventional face cleaning brushes still exhibit the following phenomena in actual uses. First, the surface profile of the bristles is fixed, so when the bristles are fixed, the contact angle of the surface profile with respect to the user's face is also fixed. However, the face shapes of different people are different and different parts of the face also have different contours. Thus, when vibrating at a high speed, the fixed bristles may make water or the facial cleanser splash, which makes the surrounding not clean. Also, if the user is not good at controlling the strength of pressing the bristles to the skin and applies a force that is too strong, the skin may be damaged due to over-stimulation. Also, due to the different contact angles between the bristles and the face, it is difficult to have the expected effect of cleaning.

Based on above, how to increase the contact area between the bristles of the face cleaning brush and the user's face so that the contact area is not reduced as the position differs should be an issue for relevant researches to work on.

### SUMMARY OF THE DISCLOSURE

The disclosure provides an electric cleaning device that improves an extent to which the electric cleaning device is fit to the user's face by utilizing a surface profile of a brush head and a relative flexibility thereof.

An embodiment of the disclosure provides an electric cleaning device, including a main body, a power source, a transmission shaft, a support stand, and a plurality of brush heads. The power source is disposed inside the main body. One end of the transmission shaft is connected to the power source, while the other end of the transmission shaft extends out of the main body and is connected to the support stand. The brush heads are flexibly assembled to the support stand and form an enclosed shape. In addition, the power source drives the brush heads to vibrate through the transmission shaft and the support stand. Each of the brush heads has a tip, and the tips form a center of the enclosed shape.

Based on above, the electric cleaning device is capable of providing a vibrating effect in different directions by using the plurality of brush heads forming the enclosed shape, so as to improve the effect of cleaning to the part that the user intends to clean. In addition, since the brush heads are flexibly disposed to the support stand, a flexibility of swinging is provided between the respective brush heads and the support stand. Therefore, when the device is used, the brush heads are able to be more fittingly adaptable to the user's face. In other words, the flexible brush heads are adaptable to different parts of the face.

### BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.
FIG. 1 is a schematic view illustrating an electric cleaning device according to an embodiment of the disclosure.
FIGs. 2 and 3 are exploded views illustrating the electric cleaning device shown in FIG. 1 from different perspectives.
FIG. 4 is a front view illustrating the power source shown in FIG. 2.
FIG. 5 is a partial cross-sectional view illustrating the electric cleaning device shown in FIG. 1.
FIG. 6 is a schematic view illustrating an electric cleaning device corresponding to parts to be cleaned of a human being's face.
FIG. 7 is a schematic front view illustrating a brush head.

### DESCRIPTION OF THE EMBODIMENTS

Reference will now be made in detail to the present preferred embodiments of the disclosure, examples of which are illustrated in the accompanying drawings. Wherever possible, the same reference numbers are used in the drawings and the description to refer to the same or like parts.

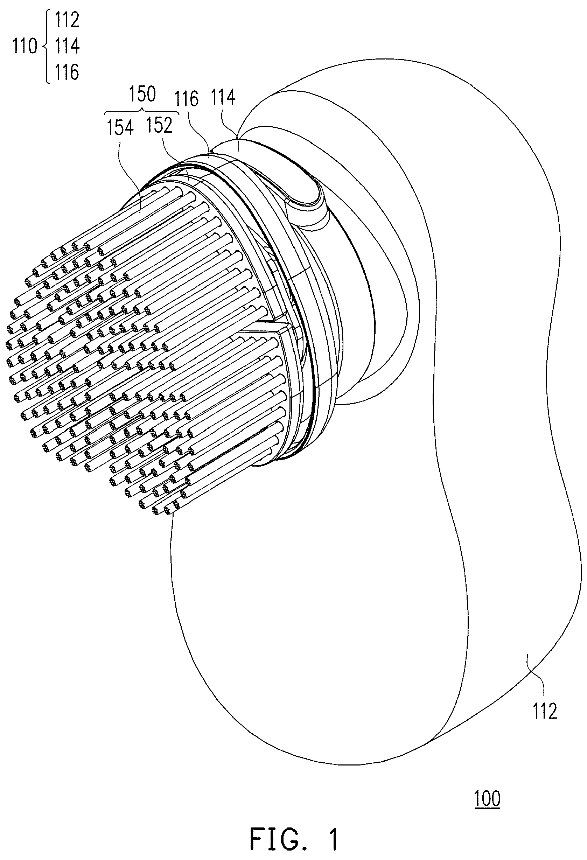

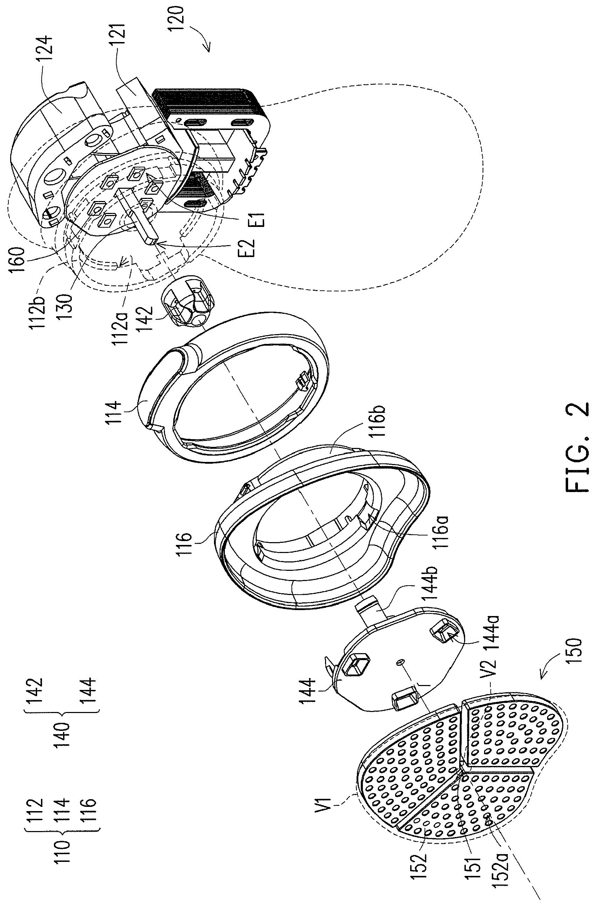

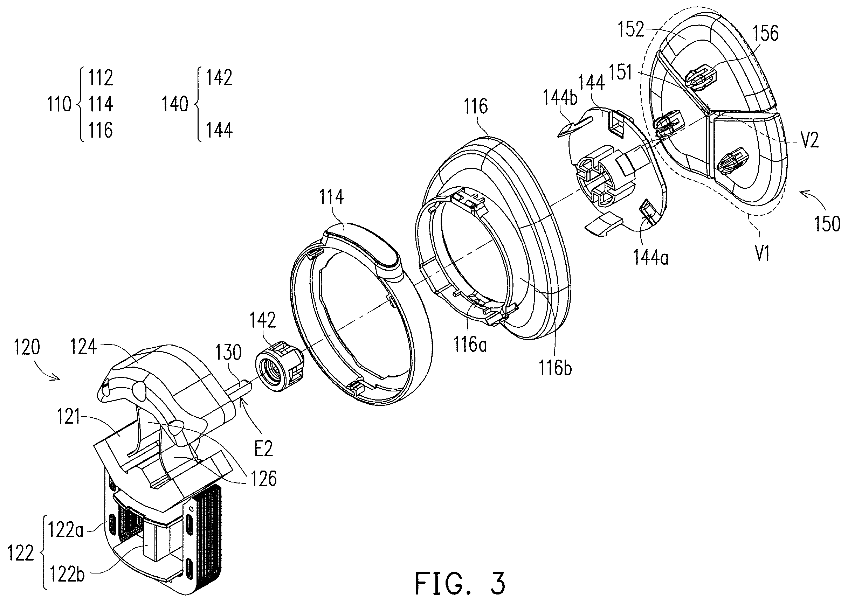

FIG. 1 is a schematic view illustrating an electric cleaning device according to an embodiment of the disclosure. FIGs. 2 and 3 are exploded views illustrating the electric cleaning device shown in FIG. 1 from different perspectives. Here, FIG. 2 illustrates a portion of a main body with broken lines, so as to illustrate connection relations of relevant components. Referring FIGs. 1 to 3 at the same time, in this embodiment, an electric cleaning device 100 includes a main body 110, a power source 120, a transmission shaft 130, a support stand 140, and a plurality of brush heads 150. The main body 110 includes a holding part 112, a connection part 114, and a basin-like outer cover 116. In addition, the connection part 114 is assembled between an end of the holding part 112 and the basin-like outer cover 116. Substantially, the connection part 114 is coupled to an outer ring 112b of the holding part 112, and the basin-like outer cover 116 is coupled to an inner edge of the connection part 114 by using a coupling part 116b of the basin-like outer cover 116. The power source 120 is disposed inside the holding part 112 of the main body 110. The transmission shaft 130 is in a bent shape. A first end E1 of the transmission shaft 130 is connected to the power source 120, and a second end E2 of the transmission shaft 130 is further inserted through the connection part 114 and the basin-like outer cover 116 after extending out of the holding part 112 through an opening 112a. The support stand 140 is connected to the second end E2 of the transmission shaft 130 and accommodated in the basin-like outer cover 116. The brush heads 150 are respectively flexibly assembled to the support stand 140 and jointly form an enclosed shape VI. In addition, a power generated by the power source 120 drives the brush heads 150 to vibrate through the transmission shaft 130 and the support stand 140. Accordingly, the use may clean his/her face by using the vibrating brush heads 150.

As shown in the figures, in this embodiment, three brush heads 150 are assembled to the support stand 140 to form the enclosed shape VI. In addition, each of the brush heads 150 has a tip 151. After assembling of the brush heads 150, the tips 151 form a center V2 of the enclosed shape V1. Namely, the brush heads 150 are assembled to the support stand 140 and divided up in a sector-like configuration. However, the disclosure does not intend to limit the number of the brush heads 150.

In this embodiment, each of the brush heads 150 includes a base 152 and a plurality of bristles 154, and the support stand 140 includes a transmission part 142 and a support part 144. In addition, the transmission part 142 is coupled to the second end E2 of the transmission shaft 130, and the support part 144 is embedded into the transmission part 142 and located in an accommodating space of the basin-like outer cover 116. The base 152 is flexibly assembled to the support part 144 of the support stand 140, and one end of the bristle 154 is inserted into and constrained in an opening 152a of the base 152. In addition, the support part 144 is latched to a latching groove 116a of the basin-like outer cover 116 by using a hook 144b thereof, so as to serve as a connecting structure between the support part 144 of the support stand 140 and the basin-like outer cover 116 of the main body 110. In addition, the bases 152 have hooks 156 (shown in FIG. 3) opposite to the bristles 154, and the support part 144 of the support stand 140 has a plurality of latching holes 144a. The hooks 156 are flexibly latched to the corresponding latching holes 144a to assemble the brush heads 150 to the support stand 140.

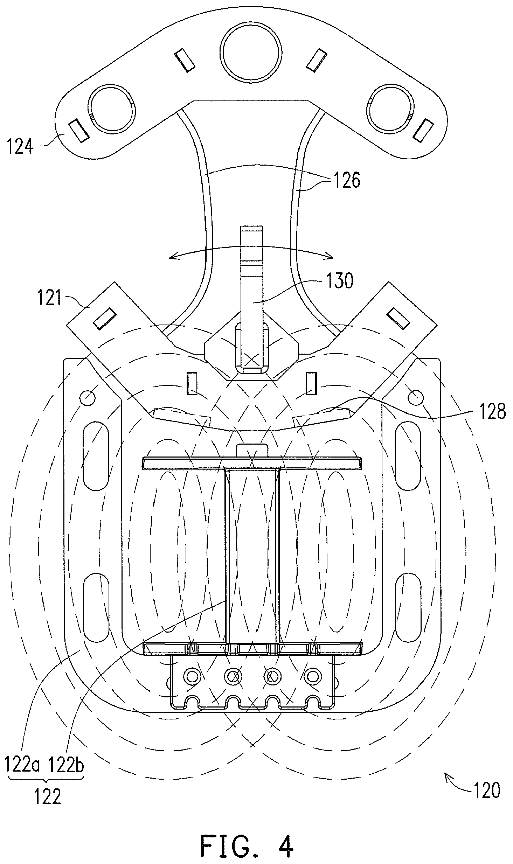

FIG. 4 is a front view illustrating the power source shown in FIG. 2. Referring to FIGs. 2 to 4 at the same time, in this embodiment, the power source 120 includes an electromagnet 122, a fixing base 124, an oscillation sheet 126, a magnet 128, and a transmission base 121. The electromagnet 122 is a ferrite core structure formed by stacking horse-shoe silicon steel plates 122a, and a magnetic line base 122b is disposed at the center of the electromagnet 122. When a current is input to the electromagnet 122, magnetic lines as shown in FIG. 4 are generated. The fixing base 124 is fixed in the body 120. The oscillation sheet 126 is a flexible steel sheet, for example. One end of the oscillation sheet 126 is fixed to the fixing base 124, and the other end of the oscillation sheet 126 is connected to the transmission base 121. The magnet 128 is disposed in the transmission base 121, and the oscillation sheet 126 and the transmission base 121 are connected at opposite sides of the magnet 128. In the meantime, opposite ends of the magnet 128 also correspond to opposite ends of the silicon steel plate 122a of the electromagnet 122.

In this configuration, when the electromagnet 122 is supplied with electricity and generates a magnetic field, the magnet 128 may swing back and forth under the influence of the magnetic lines (as shown in broken lines in the figure). Under such circumstance, the transmission base 121 may also swing back and forth accordingly, so as to make the transmission shaft 130 whose first end E1 is connected to the transmission base 121 swing (oscillate) as indicated by a bi-directional arrow sign in the figure, thereby making the brush heads 150 vibrate through the support stand 140. It should be noted that even though FIG. 4 of this embodiment illustrates two magnets 128, the number of magnets is not limited thereto. In another embodiment not shown herein, there may be only one magnet (whose shape and size are equivalent to those of the combination of the magnets 128 shown in FIG. 4) disposed on the transmission base 121.

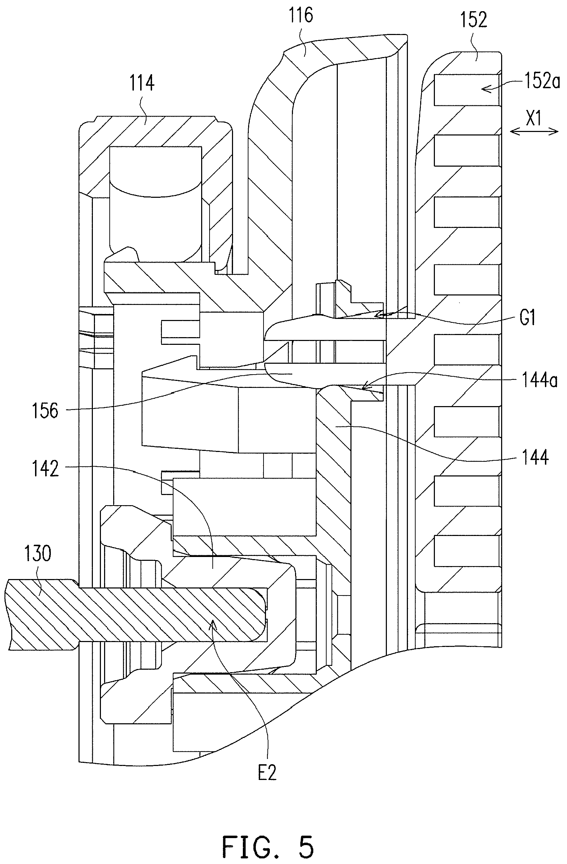

FIG. 5 is a partial cross-sectional view illustrating the electric cleaning device shown in FIG. 1. Referring to FIGs. 2, 3, and 5 together, in this embodiment, the brush head 150 is also movable with respect to the support stand 140 with a design between the hook 156 of the base 152 and the latching hole 144a of the support stand 140 utilizing a size difference of a latching structure.

More specifically, a size of the hook 156 along an assembling direction is substantially greater than a size of the latching hole 144a along the assembling direction, and there is a gap G1 between the hook 156 and the latching hole 144a. In addition, the assembling direction is parallel to an extending direction of the bristles 154, i.e., parallel to a direction of an axis X1 in the figure. In the meantime, the base 152 of this embodiment is elastic, and is formed of materials such as silicone rubber, for example, so as to allow the base 152 to swing with respect to the support part 144 of the support stand 140. In addition, since the hole 144a has a shape that expands outward toward the base 152, the base 152 of the brush head 150 is provided with an additional flexibility of swinging when driven by the support stand 140 to oscillate.

Based on above, by utilizing the material property (being elastic) of the base 152 and a property of the connection structure between the hook 156 of the base 152 and the latching hole 144a of the support part 144, the brush head 150 of this embodiment is further provided with the additional flexibility of swinging when driven by the support stand 140, so that the bristles 154 is fittingly adaptable to the user's facial contour. Thus, even if the bristles 154 contact different parts of the face, a contact area between the bristles 154 and the face is still maintained. In other words, with the movable structure of the brush head 150, an adaptability of the electric cleaning device 100 of this embodiment to the facial contour is increased. For example, when the three brush heads 150 of this embodiment contact the user's face, there maybe different relative distances and deformation states between the respective brush heads 150 and the support stand 140 in correspondence to the user's facial contour, so as to ensure that the bristles 154 of the brush heads 150 are able to effectively contact the surface of the face.

In addition, in an unstressed condition, the base 152 of the brush head 150 also keeps a distance from a circumference the basin-like outer cover 116, so as to provide a space for the base 152 to deform or move in correspondence with the facial contour.

Referring to FIG. 2 again, in this embodiment, the electric cleaning device 100 further includes a plurality of light sources 160, such as a plurality of light emitting diodes. The light sources 160 are disposed between the main body 110 and the power source 120, surround the transmission shaft 130, and are electrically connected to an electric power source (not shown herein, but the electric power source may be the same electric power source as required by the power source 120 of this embodiment) disposed inside the main body 110. Besides, the support stand 144 and the base 152 of the brush head 150 are light-transmissive. Thus, light may be emitted out of a space between the bristles 154 through the support stand 144 and the base 152. Accordingly, the light sources 160 may provide a sterilizing light beam (e.g., ultraviolet light), so as to provide a facilitated effect of cleaning to the user. Here, positions where the light sources 160 are disposed are not limited. The light sources 160 may also be disposed inside the main body 110 and face the brush heads 150 to provide the light emitted out of the brush heads 150.

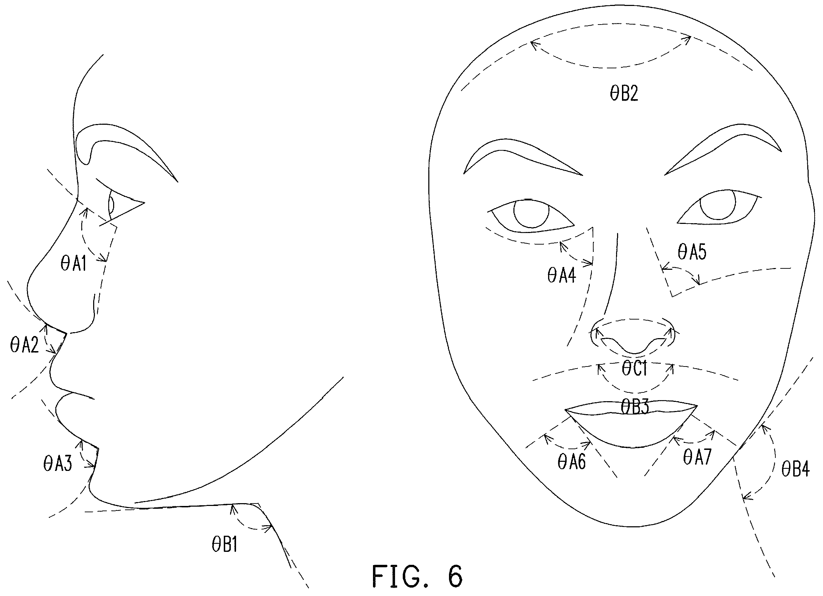

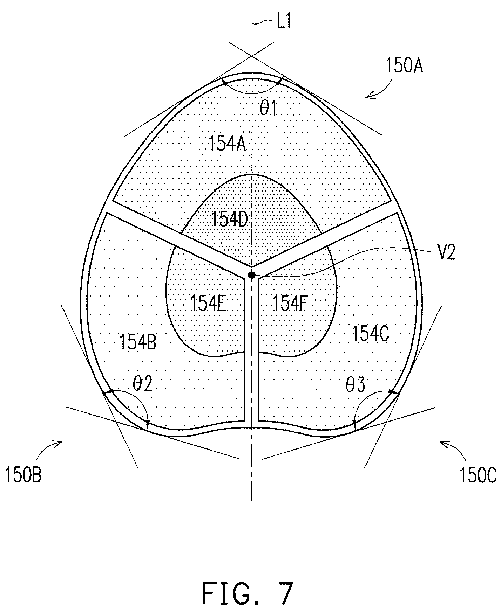

FIG. 6 is a schematic view illustrating an electric cleaning device corresponding to parts to be cleaned of a human being's face. FIG. 7 is a schematic front view illustrating a brush head. Referring to FIGs. 6 and 7 at the same time, the electric cleaning device 100 includes three brush heads, namely a first brush head 150A, a second brush head 150B, and a third brush head 150C. The three brush heads 150A, 150B, and 150C are substantially in a triangular arrangement, wherein an arc angle θ1 of an outer edge of the first brush head 150A is smaller than an arc angle θ2 of an outer edge of the second brush head 150B and an arc angle θ3 of an outer edge of the third brush head 150C, while the arc angles θ2 and θ3 of the outer edges of the second brush head 150B and the third brush head 150C are equal to each other and symmetrical to each other with respect to a central axis LI passing through a center V2 of the enclosed shape V1.

Accordingly, by designing the arc angle θ1 to be different from the arc angles θ2 and θ3, a part of a human being's face that the first brush head 150A is able to correspondingly clean is also different from parts that the second brush head 150B and the third brush head 150C are able to clean. Referring to parts of a human face shown in FIG. 6, some parts have smaller flare angles, such as parts with flare angles θA1 to θA7. The flare angles of these parts are approximately from 75 degrees to 120 degrees. Thus, these parts may be cleaned with the first brush head 150A having a smaller arc angle. As for parts with flare angles θB1 to θB4, these parts have greater flare angles of approximately 125 degrees or more. Thus, these parts are suitable to be cleaned with the second brush head 150B and the third brush head 150C having greater arc angles.

Also, a hardness of bristles 154A and 154D disposed on the first brush head 150A is smaller than a hardness of bristles 154B and 154E disposed on the second brush head 150B and a hardness of bristles 154C and 154F disposed on the third brush head 150C. Thus, when corresponding applied to the parts of the human being's face, the first brush head 150A with the softer bristles 154A and 154D may clean the parts with the smaller flare angles θA1 to θA7, and the softer bristles 154A and 154D are consequently able to flexibly clean deeper. On the contrary, for those with greater arc angles, such as the parts with the flare angles θB1 to θB4, since the contour of these parts is flatter, the bristles 154B and 154E and the bristles 154C and 154F that are harder may perform large-area cleaning.

Besides, the bristles of the respective brush heads may be further distinguished. Since the light sources 160 may provide the sterilizing light emitted out of the brush heads, in this embodiment, the brush head 150A further includes the bristles 154A and 154D, wherein the bristles 154D are located beside the center V2 of the enclosed shape V1, and the bristles 154A are away from the center V2 of the enclosed shape V1 and surround the bristles 154D. More importantly, the bristles 154D are formed of a light-transmissive material, while the bristles 154A are formed of an opaque material, and the light sources 160 substantially correspond to the bristles 154D. Accordingly, the light of the light source may pass through the bristles 154D of the brush head 154A. Similarly, the second brush head 150B also has the light-transmissive bristles 154E and the opaque bristles 154B, and the third brush head 150C has the light-transmissive bristles 154F and the opaque bristles 154C. After the brush heads 150A, 150B, and 150C are combined, the light-transmissive bristles 154D, 154E, and 154F jointly form an enclosed profile, while the opaque bristles 154A, 154B, and 154C jointly form an enclosed hollow profile and surround the light-transmissive bristles 154D, 154E, and 154F. Accordingly, the light-transmissive bristles 154D, 154E, and 154F located at the inner are able to clean the part with a flare angle of θC1 on the human being's face by using the light of the light sources 160, such as the nose, so as to effectively clean the part by using the sterilizing light, such as cleaning blackheads on the nose. However, the embodiment does not intend to limit a radiatable range of the light sources 160. The range may be suitably adjusted based on conditions of cleaning as required.

In view of the foregoing, in the embodiments of the disclosure, the electric cleaning device is capable of providing a vibrating effect in different directions by using the plurality of brush heads forming the enclosed shape, so as to improve the effect of cleaning to the part that the user intends to clean. In addition, the connection structure between the bases of the brush heads and the support part of the support stand, i.e., the size difference and the gap between the hooks and the latching holes, allows the base to provide an additional flexibility of swinging in addition to the movement driven by the support part. In the meantime, since the base is elastic, when the bristles touch the user's face, there may be different states of deformation in different states or relative positions between the base and the support stand may be changed due to differences in force applied or surface contours. In this way, the brush heads may be fittingly adaptable to the user's face in correspondence with the user's facial contour.

Besides, since the base of the brush heads are light-transmissive, the light provided by the light sources disposed in the main body may be emitted out of the space between the bristles through the base, so as to facilitate the effect of cleaning in actual use.

## Claims

1. An electric cleaning (100) device for cleaning a human being's face, the device comprising:
a main body (110);
a power source (120), disposed inside the main body (110);
a transmission shaft (130), wherein one end (E1) of the transmission shaft (130) is connected to the power source (120), and the other end (E2) of the transmission shaft (130) extends out of the main body (110);
a support stand (140), connected to the other end (E2) of the transmission shaft (130); and
a plurality of brush heads (150), flexibly assembled to the support stand (140) to form an enclosed shape (V1), wherein the power source (120) drives the brush heads (150) to vibrate through the transmission shaft (130) and the support stand (140), each of the brush heads (150) has a tip (151), and the tips (151) form a center (V2) of the enclosed shape (VI).

2. The electric cleaning device (100) as claimed in claim 1, wherein each of the brush heads (150) comprises:
a base (152), assembled to the support stand (140) and keeping a gap (G1) from the support stand (140); and
a plurality of bristles (154), disposed on the base (152) and located at an opposite side of the support stand (140).

3. The electric cleaning device (100) as claimed in claim 2, wherein each of the bases (152) has a hook (156) opposite to the bristles (154), the support stand (140) has a plurality of latching holes (144a), and the hooks (156) are flexibly latched to the corresponding latching holes (144a), so that the brush head (150) is assembled to the support stand (140).

4. The electric cleaning device (100) as claimed in claim 3, wherein a size of the hook (156) along an assembling direction is greater than a size of the latching hole (144a) along the assembling direction, the gap (G1) is between the latching hole (144a) and the hook (156), and the assembling direction is parallel to an extending direction of the bristles (154).

5. The electric cleaning device (100) as claimed in claim 3, wherein each of the latching holes (144a) has a profile expanding outward toward the base (152).

6. The electric cleaning device (100) as claimed in claim 3, wherein the support stand (140) comprises:
a transmission part (142), coupled to the other end (E2) of the transmission shaft (130); and
a support part (144), embedded to the transmission part (142), wherein the support part (144) has the latching holes (144a).

7. The electric cleaning device (100) as claimed in claim 2, wherein the base (152) is elastic.

8. The electric cleaning device (100) as claimed in claim 2, wherein the base (152) is light-transmissive.

9. The electric cleaning device (100) as claimed in claim 2, further comprising:
a plurality of light sources (160), disposed between the main body (110) and the power source (120), wherein the light sources (160) surround the transmission shaft (130) to provide light that is emitted out of the bristles (154) through the support stand (144) and the base (152).

10. The electric cleaning device as claimed in claim 2, wherein the main body (110) has a basin-like outer cover (116) toward the brush heads (150), and each of the bases (152) keeps a distance from a circumference of the basin-like outer cover (116).

11. The electric cleaning device (100) as claimed in claim 2, comprising a first brush head (150A), a second brush head (150B), and a third brush head (150C), wherein an arc angle (θ1) of an outer edge of the first brush head (150A) is smaller than arc angles (θ2) of outer edges of the second brush head (150B) and the third brush head (150C), and the arc angles (θ3) of the outer edges of the second brush head (150B) and the third brush head (150C) are equal to each other and symmetrical to each other with respect to a central axis (L1) passing through the center (V2) of the enclosed shape (V1).

12. The electric cleaning device (100) as claimed in claim 11, wherein each of the brush heads (150A, 105B, 150C) comprises a plurality of first bristles (154D, 154E, 154F) and a plurality of second bristles (154A, 154B, 154C), the first bristles (154D, 154E, 154F) are beside the center (V2) of the enclosed shape (V1), the second bristles (154A, 154B, 154C) are away from the center (V2) of the enclosed shape (V1) and surround the first bristles (154D, 154E, 154F), the first bristles (154D, 154E, 154F) are formed of a light-transmissive material, and the second bristles (154A, 154B, 154C) are formed of an opaque material.

13. The electric cleaning device as claimed in claim 11, wherein a hardness of the bristles (154A, 154D) disposed on the first brush head (150A) is lower than a hardness of the bristles (154B, 154E) disposed on the second brush head (150B) and a hardness of the bristles (154C, 154F) disposed on the third brush head (150C).
